# EUROPEAN PATENT SPECIFICATION

(11) **EP 2 196 470 B1**
(45) Date of publication and mention of the grant of the patent: **05.06.2013**
(21) Application number: 08830569.3
(22) Date of filing: 05.09.2008
(51) Int. Cl.: C07K 1/14

(54) **METHODS FOR SEPARATING RECOMBINANT PROTEINS IN AQUEOUS TWO-PHASE SYSTEMS**
VERFAHREN ZUR TRENNUNG REKOMBINANTER PROTEINE IN WÄSSRIGEN ZWEIPHASENSYSTEMEN
PROCÉDÉS DE SÉPARATION DE PROTÉINES RECOMBINANTES DANS DES SYSTÈMES AQUEUX BIPHASÉS

(30) Priority: 12.09.2007 ES 200702504
(43) Date of publication of application: 16.06.2010
(73) Proprietor: Biomedal, S.L., 41092 Sevilla (ES)
(72) Inventor: SANZ MORALES, Jesús, Miguel, E-41092 Sevilla (ES); MAESTRO GARCIA-DONAS, Beatriz, E-41092 Sevilla (ES); AREVALO RODRIGUEZ, Miguel, E-41092 Sevillla (ES); VELASCO UMPIERREZ, Isabel, E-41092 Sevilla (ES); CEBOLLA RAMIREZ, Angel, E-41092 Sevilla (ES)
(74) Representative: Temino Ceniceros, Ignacio
(86) International application number: PCT/ES2008/000578
(87) International publication number: WO 2009/034203

(56) References cited:
- WO-A1-00/58342
- FEXBY S ET AL: "Hydrophobic peptide tags as tools in bioseparation", TRENDS IN BIOTECHNOLOGY, ELSEVIER PUBLICATIONS, CAMBRIDGE, GB, vol. 22, no. 10, 1 October 2004 (2004-10-01), pages 511-516, XP004575228, ISSN: 0167-7799, DOI: DOI:10.1016/J.TIBTECH.2004.08.005
- KOELLNER G. ET AL.: 'A neutral molecule in a cation-binding site: specific binding of a PEG-SH to acetylcholinesterase from torpedo californica' J.MOL.BIOL. vol. 320, 2000, pages 721 - 725, XP008132090
- GUAN Y. ET AL.: 'New approaches to aqueous polymer systems:theory,thermodynamics and applications to biomolecular separations' PURE&APPL.CHEM vol. 67, no. 6, 1995, pages 955 - 962, XP008132169

## Description

### FIELD OF THE INVENTION

The present invention refers to a procedure for separating polypeptides or proteins, fused to a choline-binding polypeptidic sequence with affinity for a water-soluble polymer, which drives partitioning of the polypeptide or protein of interest to the phase rich in said soluble polymer, in two-phase aqueous systems containing it. The procedure allows a fast separation of the fusion protein from a complex protein mixture present in a cell extract, in a simple process that can be used for the purification of the polypeptide or protein of interest. The purification process includes one or more washing steps and concludes with an inversion in the distribution of the fusion protein induced by addition of choline to the system. The field of application of this invention therefore comprises the production, separation and eventual purification of polypeptides and proteins of biotechnological interest, with industrial, agronomic, therapeutic or diagnostic purposes in biomedicine, or as a research and analysis tool in the field of life sciences.

### STATE OF THE ART

Immobilization in solid supports is a usual step during protein purification and setting-up of enzymatic reactors for all kinds of biotransformations (Mosbach, K., ed. Immobilized Enzymes and Cells Part B. Methods in Enzymology. Vol. 135. 1987). Thus, a wide variety of immobilization systems have been developed, which are based on either covalent or non-covalent interactions between the protein and the solid support. In some cases, expression of the protein of interest as a fusion with an affinity polypeptide or "tag" facilitates immobilization of the protein through a strong and specific interaction with the solid support (Uhlen, M., et al., Fusion proteins in biotechnology. Curr Opin Biotechnol, 1992. 3(4): p. 363-9; Waugh, D.S., Making the most of affinity tags. Trends Biotechnol, 2005. 23(6): p. 316-20). However, immobilization on solid matrices often results in protein structural changes with negative effects at the functional level. In addition, the physical and chemical properties of the support can affect mass transfer (substrate access to the immobilized enzyme, and diffusion of the products to the mobile phase), causing changes in the catalyst kinetic constants that may reduce process efficiency. Moreover, industrial scale-up of separation procedures, based on the use of solid supports in chromatography columns, often require high working pressures and are thus prone to technical difficulties resulting from the mechanical limitations of the materials and structures involved.
As an alternative to the separation procedures based on solid supports, liquid systems have been developed that involve partitioning of the polypeptide or protein of interest in a two-phase aqueous solution. Aqueous two-phase systems generate spontaneously in solutions containing mixtures of two structurally different polymers, or a polymer (usually polyethylenglycol, PEG) and a concentrated salt (Johansson, H.W.a.G., ed. Aqueous Two-Phase Systems. Methods in Enzymology. Vol. 228. Guan, Y. et al, New approaches to aqueous polymer systems: Theory, thermodynamics and applications to bimolecular separations. Pure and Applied Chemistry, 1995. 67(6): p. 955-962; Andrews, B.A., A.S. Schmidt, and J.A. Asenjo, Correlation for the partition behavior of proteins in aqueous two-phase systems: effect of surface hydrophobicity and charge ,Biotechnol Bioeng, 2005. 90(3): p. 380-90). In two-phase systems composed of two polymers, each phase is enriched in one of the polymers, while in polymer-salt two-phase systems one phase is enriched in the polymer, and the other in the salt. The chemical and physical properties of these mixtures have been extensively studied (Cabezas, H., Jr., Theory of phase formation in aqueous two-phase systems. J Chromatogr B Biomed Appl, 1996. 680(1-2): p. 3-30) and binodal diagrams have been obtained that describe the concentration ranges of the components that result in phase separation. In these models, the so-called "tie lines" predict the final concentration of each polymer/salt in each phase, as well as their relative volumes (Johansson, H.W.a.G., ed. Aqueous Two-Phase Systems. Methods in Enzymology. Vol. 228. 1994). Aqueous two-phase systems have shown to be very useful in the separation of biological material (Hustedt, H., K.H. Kroner, and M.R. Kula, Applications of phase partitioning in biotechnology., in Partitioning in Aqueous two-phase systems: theory, methods uses and applications in biotechology, H. Walter, D.E. Brooks, and D. Fisher, Editors. 1985, Academic Press: Orlando, FL,. p. 529-587; Persson, J., et al., Purification of recombinant and human apolipoprotein A-1 using surfactant micelles in aqueous two-phase systems: recycling of thermoseparating polymer and surfactant with temperature-induced phase separation. Biotechnol Bioeng, 1999. 65(4): p. 371-81). Aqueous two-phase systems present several advantages, such as high biocompatibility, low surface tension (minimizing biomolecule degradation), high loading capacity and yield, and easy scaling-up (Albertsson, P.A., Partition of cell particles and macromolecules. 1986, New York: Wiley; Walter, H., G. Johansson, and D.E. Brooks, Partitioning in aqueous two-phase systems: recent results. Anal Biochem, 1991. 197(1): p. 1-18). In addition, mass transfer in aqueous two-phase systems is more efficient than in solid support based systems, and they are economic and flexible, being easily adapted for the partition of different molecules of interest. For a given protein, partitioning between two particular phases depends on a combination of factors such as the charge, size and hydrophobicity of the protein in question, and in general is poorly predictable, a limitation that has hampered a major establishment of two-phase systems in the market. A variation of the two-phase system involves the use of affinity polypeptides or "tags" fused to the protein of interest, to promote its partitioning to one of the phases. This new system for partitioning by affinity in aqueous two-phase systems has shown to be more predictable, and promising results have been obtained using the widely used hexahistidine "tag" (Chung, B.H., D. Bailey, and F.H. Arnold, Metal affinity partitioning., in Aqueous Two-Phase Systems, H.W.a.G. Johansson, Editor. 1994. p. 167-277; Birkenmeier, G., et al., Immobilized metal ion affinity partitioning, a method combining metal-protein interaction and partitioning of proteins in aqueous two-phase systems. J Chromatogr, 1991. 539(2): p. 267-77). In this system, the His-tagged protein partitions to a phase rich in PEG that has been previously derivatized with metal ions. Similarly, tyrosine or tryptophan-rich sequences have been successfully used as polypeptide tags in this kind of affinity partitioning procedures (Fexby, S., et al., Partitioning and characterization of tyrosine-tagged green fluorescent proteins in aqueous two-phase systems. Biotechnol Prog, 2004. 20(3): p. 793-8; Collen, A., et al., Genetic engineering of the Trichoderma reesei endoglucanase I (Cel7B) for enhanced partitioning in aqueous two-phase systems containing thermoseparating ethylene oxide--propylene oxide copolymers. J Biotechnol, 2001. 87(2): p. 179-91).

The choline-binding modules (CBM) constitute a family of polypeptides found in choline-binding proteins (CBP), present in a variety of microorganisms (Swiatlo, E. et. al., Choline-binding proteins., in The Pneumococcus, E.I. Tuomanen, T.J. Mitchell, and D.A. Morrison, Editors. 2004, American Society for Microbiology: Washington, DC. p. 49-60). CBMs contain the so-called choline-binding repeats (CBR; code Pfam PF01473: http://www.sanger.ac.uk//cgi-bin/Pfam/getacc?PF01473), which are highly conserved, 20 amino acid-long sequences that form secondary structures of the loop-β-hairpin type (Fernandez-Tornero, C., et al., A novel solenoid fold in the cell wall anchoring domain of the pneumococcal virulence factor LytA. Nat Struct Biol, 2001. 8(12): p. 1020-4; Hermoso, J.A., et al., Insights into pneumococcal pathogenesis from the crystal structure of the modular teichoic acid phosphorylcholine esterase Pce. Nat Struct Mol Biol, 2005. 12(6): p. 533-8; Hermoso, J.A., et al., Structural basis for selective recognition of pneumococcal cell wall by modular endolysin from phage Cp-1. Structure, 2003. 11(10): p. 1239-49). Two consecutive CBRs configure a binding site for choline. The affinity of the CBMs to choline and structural analogs of it (Sanz, J.M., R. Lopez, and J.L. Garcia, Structural requirements of choline derivatives for 'conversion' of pneumococcal amidase. A new single-step procedure for purification of this autolysin. FEBS Lett, 1988. 232(2): p. 308-12), has allowed the development of a system for efficient purification of proteins fused with some of these CBMs by affinity chromatography (Sanchez-Puelles, J.M., et al., Immobilization and single-step purification of fusion proteins using DEAE-cellulose. Eur J Biochem, 1992. 203(1-2): p. 153-9). Basically, the procedure consists on applying a cell extract, containing the fusion protein, to a support derivatized with tertiary or quaternary amines, such as the diethylaminoethanol (DEAE). The protein thus immobilized maintains its functionality and can be easily eluted through the addition of a competitor ligand, such as choline. This procedure is the base of two previous patents (ES 2 032 717 and ES200700281) in which the CBM derives from the C-terminal domain of the LytA amidase and is named LYTAG. Among the advantages of this procedure, it is worth mentioning that the system is highly specific, can be used with a wide variety of already commercially available supports (e.g., chromatographic resins, paper, multiwell plates), shows very few incompatibilities and all the advantages desirable in an industrial scalable system, as the use of economic, non toxic materials and compounds. The object of the present invention comprises a protocol for the partitioning of polypeptides and proteins, fused to the LYTAG module, in aqueous two-phase systems, said partitioning being modulated by the addition of choline. This invention expands the applications of the LYTAG module, from its current configuration as an affinity tag in solid phase affinity chromatography, to its use in affinity partitioning in aqueous two-phase systems. Furthermore, the procedure can employ any choline-binding module of the same structural family than LYTAG.

### BRIEF DESCRIPTION OF THE INVENTION

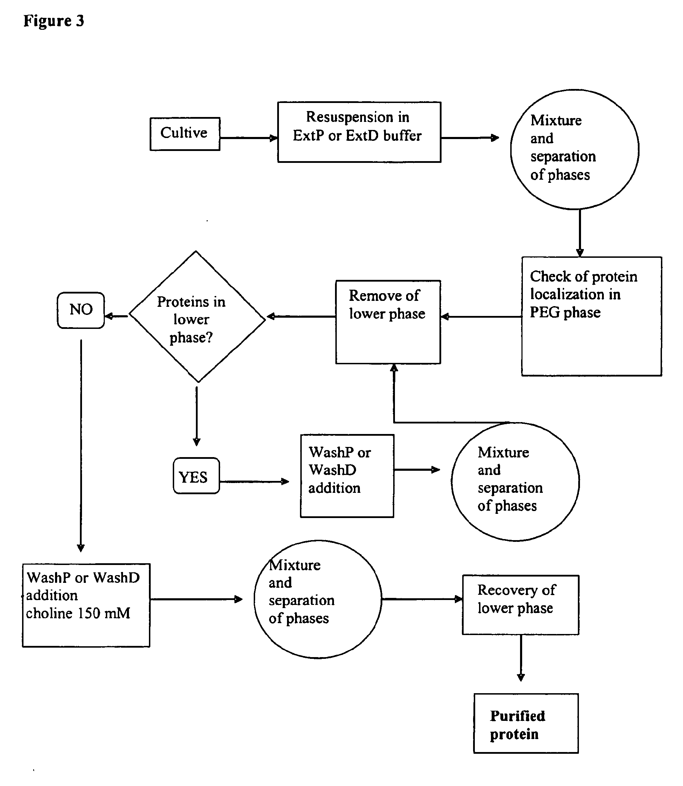

The object of the present invention is a procedure for solution partitioning and purification of polypeptides and proteins fused to choline-binding polypeptidic modules, based on the use of binary aqueous systems in which the main compounds are distributed spontaneously in two phases, one of them rich in a soluble polymer with affinity to choline-binding modules (CBMs) and the other one enriched in a different polymer or in a salt. The procedure follows the scheme showed in Fig 3, and it consists basically in the incubation of an aqueous extract, obtained from a cell culture that expresses a fusion of the protein of interest with a choline-binding module (CBM) containing at least one choline-binding repeat (CBR), with the binary system components in the appropriate concentrations and proportions for favouring the separation of the mixture in two differentiated phases. As a result of the CBM affinity to the above mentioned polymer, the fusion protein is located after phase separation preferably in the phase rich in this polymer, so that it is possible to remove the opposite phase, and concomitantly, the other proteins and cellular components from the extract that have partitioned to it. The discarded phase is then substituted with an equivalent volume of a fresh solution with the same original composition, and the system is mixed and then allowed to settle for phase separation, before discarding the same phase as in the previous step. This washing procedure is repeated as many times as needed, until the removed phase presents a minimum quantity of contaminant proteins. At this point, the discarded phase is replaced with an equivalent volume of a solution with the same original composition, this time containing choline at a concentration that has the effect of reverting the binding of the CBM-tagged protein to the polymer, competing with this polymer for the binding to the protein and specifically displacing it to the opposite phase, from where it can be finally recovered with a high purity grade.

The procedure object of the present invention comprises preferably the use of PEG (3-15%) and potassium phosphate (5-15%), for example at 15% and 12-5%, respectively. In another preferred configuration potassium phosphate can be substituted with another, highly soluble phosphate, sulphate or citrate salt, which is compatible with formation of the two-phase system. The PEG molecular weight useful in this system ranges from PEG-6000 to PEG-20000, being the preferred configuration the one that uses PEG-8000 (henceforth, PEG).

In another preferred configuration, the object of the present invention comprises the use of PEG (3-15%) and dextran (5-10%, for example at 6%) dissolved in 20 mM Tris-HCl buffer pH 8.0. In another preferred configuration, dextran can be replaced with dextran derivatives, or with starch or other polysaccharides with a similar structure, or with their derivatives.

Depending on the two-phase system chosen, the cells from the microbial culture are harvested by centrifugation and resuspended preferably in 20 mM potassium phosphate buffer pH 8.0, if the PEG/Phosphate system is used, or in 20 mM Tris-HCl buffer pH 8.0 in the case of the PEG/Dextran system. After lysing the cells through sonication or other procedure, the resulting crude extract is centrifuged to remove the cell debris, and a sufficient amount of PEG and dipotassium phosphate, or of PEG and dextran, is added to the supernatant to obtain the concentrations and proportions described for each system. The solution thus obtained is gently mixed and then allowed to separate in two phases. Phase separation can be accelerated by centrifugation. Partitioning of the CBM-fused polypeptide or protein to the upper, PEG-rich phase can be verified by electrophoretic analysis in polyacrilamide gels (SDS-PAGE), followed by Coomassie Blue staining, or by immunological methods using antibodies that recognize the choline-binding module, or the protein of interest, if available. Next, the lower, phosphate- or dextran- rich phase (depending on the system of choice) is removed using a pipette or a decantation funnel, after which, the discarded phase is replaced by an equivalent volume of a solution with the same original composition, according to the binodal diagram of the system in question (Johansson, H.W.a.G., ed. Aqueous Two-Phase Systems. Methods in Enzymology. Vol. 228. 1994), with the aim of restoring the separation of the phases and their relative volumes. After repeating several times this washing process, the protein of interest can be recovered from the PEG-rich phase, or in the opposite phase after addition of choline as described above. The invention described represents an alternative to the use of solid chromatographic supports for the purification of proteins produced by recombinant expression.

### DETAILED DESCRIPTION OF THE INVENTION

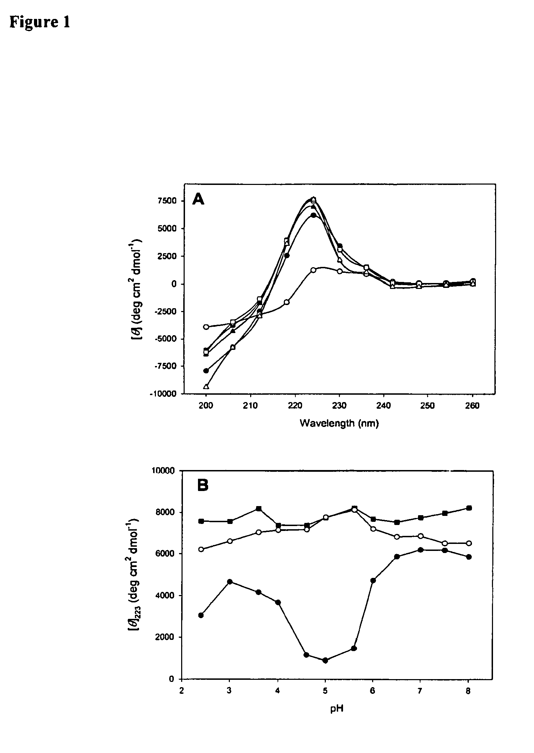

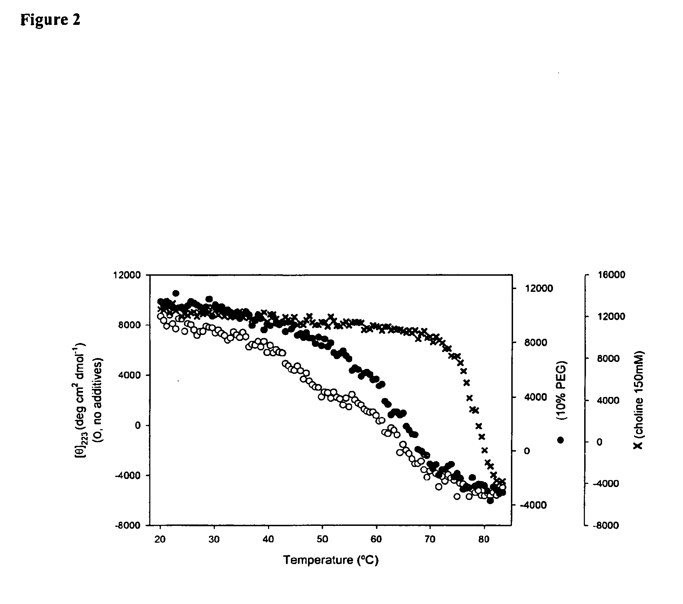

The development of the procedure object of the present invention sets off with the observation that polyethylenglycol, a polymer widely used in aqueous two-phase systems, interacts in a specific manner with the choline-binding proteins such as the acetylcholinesterase, by binding to the choline recognition site and thus acting as an inhibitor of the enzyme at high concentrations (Koellner, G., et al., A neutral molecule in a cation-binding site: specific binding of a PEG-SH to acetylcholinesterase from Torpedo californica. J Mol Biol, 2002. 320(4): p. 721-5). The choline-binding modules as the carboxyl-terminal of the protein LytA (C-LytA) from *S. pneumoniae,* the lysozyme CPL1 from the bacteriophage Cp-1, or in general, any choline-binding module of the CBM family from a variety of microorganisms (patent ES2032717; Swiatlo, E. et. al., Choline-binding proteins., in The Pneumococcus, E.I. Tuomanen, T.J. Mitchell, and D.A. Morrison, Editors. 2004, American Society for Microbiology: Washington, DC. p. 49-60), contain structural repeats with natural affinity to choline. The present invention exploits this specific interaction between PEG and these CBMs. This interaction can be revealed by circular dichroism analysis of the LYTAG module (a C-LytA derivative) in the presence of the polymer. In aqueous medium at pH 7 and the absence of choline the LYTAG protein is found partly denatured (Maestro, B. and J.M. Sanz, Accumulation of partly folded states in the equilibrium unfolding of the pneumococcal choline-binding module C-LytA. Biochem J, 2005. 387(Pt 2): p. 479-88) and as a result, its circular dichroism spectrum is poorly reproducible, and highly affected by sample storing conditions, including the freezing-thawing cycles suffered during the assays. However, addition of choline stabilizes the LYTAG protein, increasing its ellipticity (Fig. 1A). According to the LYTAG choline binding structure (Fernandez-Tornero, C., et al., A novel solenoid fold in the cell wall anchoring domain of the pneumococcal virulence factor LytA. Nat Struct Biol, 2001. 8(12): p. 1020-4), this amino alcohol seems to play an essential role in the protection of the hydrophobic zones present in the choline-binding sites, and in inducing dimerization through the last fork, resulting in a significant increase in protein stability (Fernandez-Tornero, C., et al., A novel solenoid fold in the cell wall anchoring domain of the pneumococcal virulence factor LytA. Nat Struct Biol, 2001. 8(12): p. 1020-4; Usobiaga, P., et al., Structural organization of the major autolysin from Streptococcus pneumoniae. J Biol Chem, 1996. 271(12): p. 6832-8). Addition of 10 % PEG produces a stabilizing effect on the circular dichroism spectrum similar to that induced by a high concentration of choline (Fig. 1A), indicating that both PEG and choline share interaction sites in LYTAG. On the other hand, the solubility of a given protein exhibits a minimum around the pH value corresponding to its isoelectric point, as the absence of a net electric charge favours the intermolecular aggregation mediated by interactions of the hydrophobic type. In this sense, the binding of a ligand to its cognate protein can contribute to hide hydrophobic regions present in it, thus increasing its solubility at pH values close to its isoelectric point. As shown in Fig. 1B, the circular dichroism signal at 223 nm in a preparation of the LYTAG protein remains unaltered in the range of pH values between 6.5 and 8.0, and it decreases below pH 6.0, with a minimum between pH 4.5 and pH 5.5. At pH 5.0 the spectrum shows a general decrease of the signal at all wavelengths (Fig. 1A), and a reduction of the sample absorbance is detected (data not shown). This suggests that the solubility of LYTAG has a minimum in this pH range, resulting in protein aggregation and decreasing this way the population of soluble molecules susceptible to produce a circular dichroism signal. These results agree with the expected according with the theoretical isoelectric point of the protein, 5.3, obtained with a software application (http://www.embl-heidelberg.de/cgi/pi-wrapper.pl). Addition of choline at a saturating concentration (140 mM) induces a general increase of the ellipticity and stabilizes the LYTAG protein in the range of pH values between 2.4 and 8.0 (Figs. 1A and 1B), supporting the hypothesis that binding of this ligand results in the hiding of hydrophobic sites that would be otherwise exposed, thus preventing protein aggregation. Also in support to this hypothesis, addition of low polarity additives such as 2% CHAPS or 140 mM 2,2-dimethylpropanol (DMP) also recover the circular dichroism spectrum at pH 5.0 (data not shown). When these assays are performed in the presence of 10 % PEG, the results are similar to those obtained with choline (Figs. 1A and 1B), reinforcing the hypothesis that this polymer interacts with the LYTAG protein, producing an effect that is similar to that produced by the binding of its natural ligand indicating that both PEG and choline interact with the same protein region. This is also indicated by the fact of that the LYTAG protein loses its capacity of being retained in a DEAE-cellulose column in the presence of PEG (data not shown), indicating that the PEG intercalates into the choline-binding sites, preventing further binding of the CBM to DEAE. Finally, it has also been shown that PEG could be emulating the stabilizing effect of choline over the LYTAG structure, by studying the thermal stability of the protein through circular dichroism analysis at 223 nm (Fig. 2). In the absence of a ligand, LYTAG is displays a biphasic transition originated by the accumulation of an intermediate state (Maestro, B. and J.M. Sanz, Accumulation of partly folded states in the equilibrium unfolding of the pneumococcal choline-binding module C-LytA. Biochem J, 2005. 387(Pt 2): p. 479-88). The presence of 150 mM choline prevents accumulation of such intermediate state, generating a unique sigmoidal curve as well as thermal stabilization, as described previously (Maestro, B. and J.M. Sanz, Accumulation of partly folded states in the equilibrium unfolding of the pneumococcal choline-binding module C-LytA. Biochem J, 2005. 387(Pt 2): p. 479-88). In similar assays carried out in the presence of PEG, results showed that addition of this compound causes an intermediate effect, preventing accumulation of the intermediate state without affecting the denaturalization temperature (Fig. 2). Taken together the studies described establish the affinity of the CBMs to PEG as one of the bases of the present invention.

### General protocol of purification by affinity of fusions to LYTAG in aqueous two-phase systems

The present invention is based on the results previously described, indicating interaction between LYTAG and PEG, and provides realization examples in which this polymer has been used in protein separation using two-phase aqueous systems, allowing the development of separation protocols for polypeptides and proteins fused with choline-binding modules.

In a preferred protocol, PEG/phosphate and PEG/dextran combinations are used in the two-phase aqueous systems used in the separation procedure. The PEG/phosphate system is formed at different concentration ranges of PEG (3-15%) and potassium phosphate (5-15%) at pH 8.0, with the preferred 15% PEG/12.5% phosphate combination, pH 8.0 (henceforth, ExtP buffer) showing the smallest volume difference between the PEG-rich (upper) phase and the phosphate-rich (lower) phase. On the other hand, the PEG/dextran system is formed at different concentration ranges of PEG (3-10%) and dextran (5-10%), with the preferred 6% PEG system/6% dextran combination, in 20 mM Tris-HCl buffer pH 8.0 (henceforth ExtD buffer), also showing the smallest volume difference between the PEG-rich (upper) phase and the dextran-rich (lower) phase.

The general protocol is summarized in Fig. 3 and starts by obtaining a cell extract expressing a polypeptide or protein of interest as a fusion with a CBM containing at least one CBR, in a preferred case, the LYTAG. Said extract is mixed with the compounds required for the generation of the two-phase system, and the phases allowed to separate by centrifugation, or by gravity. The LYTAG affinity to PEG described before determines the quantitative partitioning of the LYTAG fusion to the upper phase containing this polymer (see detailed examples below). Once verified the accumulation of the LYTAG fusion in the upper phase, a series of washing steps can be applied consisting in removing the lower phase and replacing it with the same volume and composition of the discarded one. Based on the data from the binodal diagrams published in the literature (Johansson, H.W.a.G., ed. Aqueous Two-Phase Systems. Methods in Enzymology. Vol. 228. 1994), and in the cases of the preferred configurations mentioned above, the composition of this fresh phase consists of 1% PEG, 16% potassium phosphate, pH 8.0 (henceforth, WashP buffer) or 0.5% PEG, 16% dextran in Tris-HCl 20 mM pH 8.0 (henceforth, WashD buffer). After a new mixing and phase separation process, the lower phase is replaced with WashP, or with WashD buffer, and the process is repeated 2-3 times. At this point, the purity of the protein of interest in the PEG phase can already be sufficiently high. However, inclusion of 150 mM choline in the WashP or the WashD buffers has the effect of displacing the PEG from the ligand-binding sites of the fusion protein, disrupting its interaction with the polymer and hence allowing recovering of the fusion protein in the lower phase, which can thus be purified to electrophoretic homogeneity.

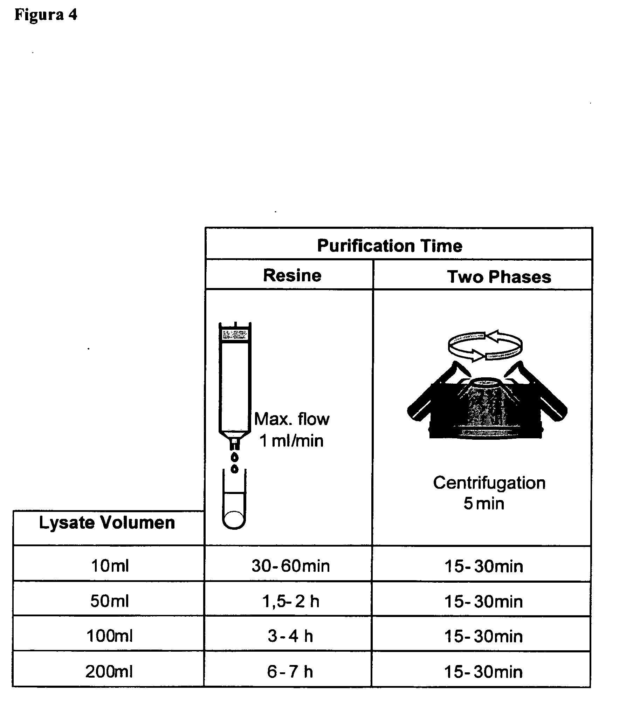

The described invention presents several advantages over other existing protein purification systems, such as being a simple, fast and economic procedure that allows purification in mild conditions. The versatility of the system is based on the wide range of reagents and reagent concentrations that can be used, allowing the adjustment to the specific needs for each particular case. Furthermore, the system is free of technical problems associated with column chromatography, such as the need for packing, pressure and clogging issues, etc., and is faster and more easily scalable than those using solid supports (Fig. 4). In addition, the absence of toxic components (such as heavy metals in the case of the systems based on the hexahistidine "tag") makes the LYTAG two-phase an environment-friendly procedure. In the described system, the absorption of the protein to the affinity compound takes place in solution, and therefore in a more efficient manner than with affinity supports based on surfaces, such as the chromatographic resins. Furthermore, the interaction of the CBM LYTAG with PEG induces stabilization of the protein in a wide pH range, opening the possibility of using these purification systems at pH values far from neutral, thus overcoming the existence of electrostatic repulsions between the protein and the chromatographic supports that takes place at acid pH values. In the examples studied, the dual affinity of the CBM for PEG and for choline seems to be the dominant criterion regarding partitioning of the LYTAG-fused protein, driving the location of a given fusion protein to the desired phase by simply adding or omitting choline from the system. Although in general it seems that the PEG/phosphate and PEG/dextran systems are *a priori* suitable for the affinity partitioning of proteins fused to CBMs as LYTAG, different features in the protein of interest, such as overall charge, size and hydrophobicity, will influence the performance of each system and hence the choice of one over the other. In this sense, the present invention is very versatile, as there are different parameters to act on for adapting the system to each particular fusion protein in order to improve partitioning, namely ionic force, temperature, pH and use of detergents, among others (Johansson, H.W.a.G., ed. Aqueous Two-Phase Systems. Methods in Enzymology. Vol. 228. 1994). Furthermore, the present invention contemplates the potential use of alternative two-phase systems such as PEG/citrate, PEG/sulphate and PEG/starch. In the case of poorly soluble proteins, extraction can be carried out in large buffer volumes, thus diluting the sample and minimizing protein aggregation and precipitation. Finally, the procedure can be integrated in enzymatic processes of biotransformation in which the enzyme can be confined in one phase or the other at will (by omitting or adding choline), while the products accumulate in the opposite phase, facilitating this way the purification of the products, minimizing the possible incidence of enzymatic inhibition by product, and recycling of the catalyst.

### FIGURES DESCRIPTION

**Figure 1**. CD analysis in the far ultraviolet of the pH effect over the LYTAG structure at 20 °C. (A), wavelength spectra registered at pH 7.0 (solid symbols) and pH 5 (open symbols) in the absence of additives (circles), in the presence of 10% PEG (triangles) or in the presence of 140 mM choline (squares). (B), pH titration followed by the CD signal at 223nm in the absence of additives (filled circles), in presence of 140 mM choline (squares) and in presence of 10 % PEG (open circles).
**Figure 2****:** Thermal stability of LYTAG followed by circular dichroism in the absence of additives (o), in presence of 10% PEG (●) and the presence of 150 mM choline (x).
**Figure 3****.** Purification of LYTAG-fused proteins by affinity partitioning.
**Figure 4****.** Comparative diagram of the purification by affinity partitioning in two-phase aqueous systems and by affinity chromatography in a solid support.

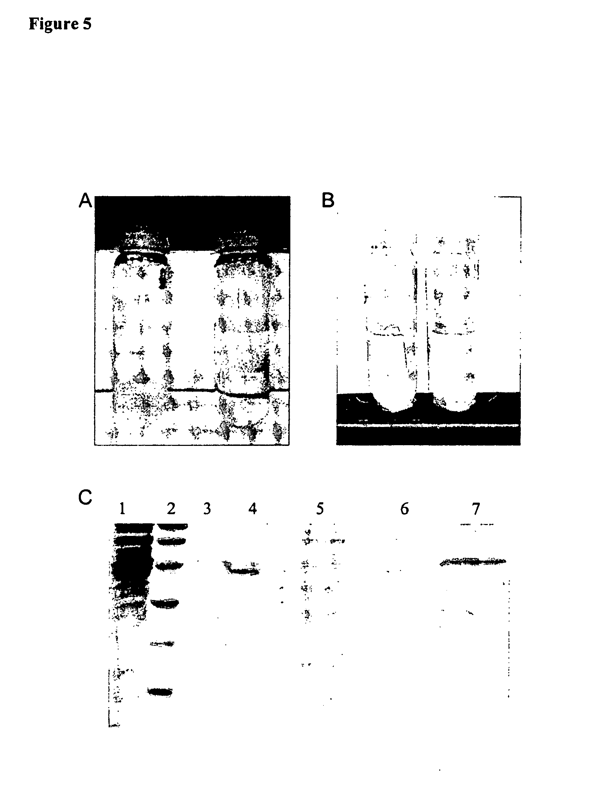

**Figure 5****.** Partitioning of the GFP-LYTAG protein in the two-phase aqueous systems PEG/dextran (left tube) and PEG/phosphate (right tube), before (A) and after (B) elution with WashD + choline and WashP + choline buffers, respectively. C), SDS-PAGE of the fraction coming from the purification in PEG/phosphate. Lane 1, Crude extract from REG-1 [pALEX2-Ca-GFP]; lane 2, Molecular weight standard; lane 3, Pure protein control, obtained by affinity chromatography; lane 4, Upper (PEG-rich) phase before elution with choline; lane 5.- Lower (phosphate-rich) phase before the elution; lane 6.-Upper phase after the elution; lane 7, Lower phase after the elution.

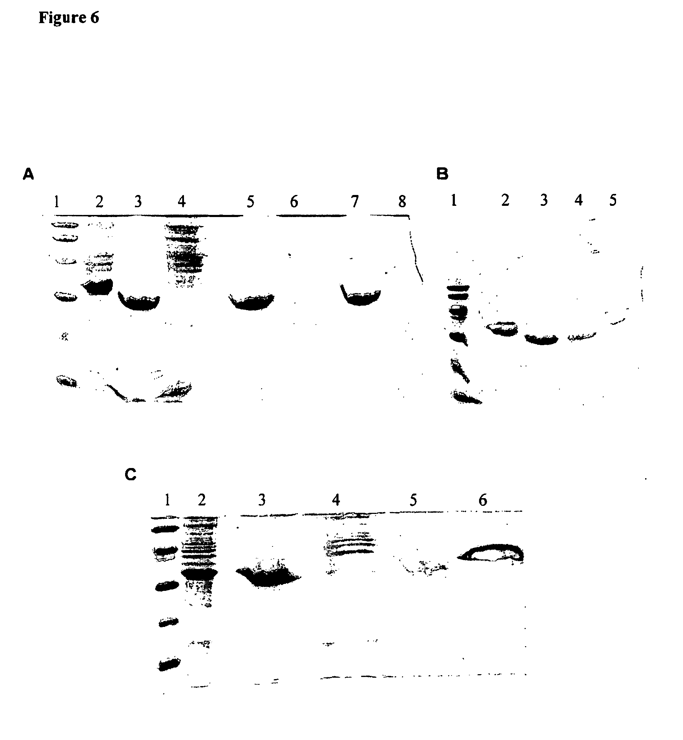

**Figure 6****.** SDS-PAGE of the fractions coming from the purification of the LYTAG-Protein A fusion in PEG/dextran. (A) lane 1, Molecular weight standard; lane 2, Crude extract of REG-1 [pALEXb-ProtA]; lane 3, Upper (PEG-rich) phase before washing; lane 4.- Lower (dextran-rich) phase before washing; lane 5.- Upper phase after the first washing; lane 6, Lower phase after the first washing; lane 7, Upper phase after the second washing; lane 8, Lower phase after the second washing. (B), lane 1, Molecular weight standard, lane 2, Upper phase after the first elution; lane 3, Lower phase after the first elution; lane 4, Upper phase after the second elution; lane 5, Lower phase after the second elution. (C), SDS-PAGE of the fractions coming from the purification in PEG/phosphate. lane 1, Molecular weight standard; lane 2, Crude extract of REG-1 [pALEXb-ProtA]; lane 3, Upper (PEG-rich) phase before elution; lane 4.- Lower (phosphate-rich) phase before elution; lane 5.- Upper phase after elution; lane 6, Lower phase after elution.

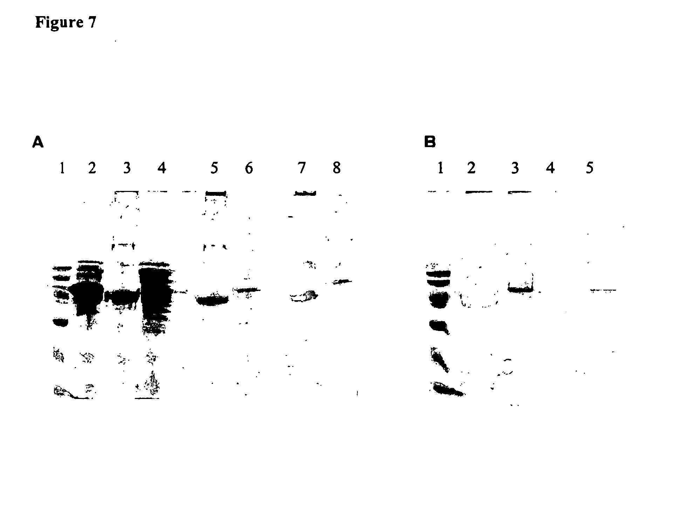

**Figure 7****.** SDS-PAGE of the fractions coming from the LYTAG-Lip36 purification in PEG/dextran. (A) lane 1, Molecular weight standard; lane 2, Crude extract of REG-1 [pALEXb-Lip36]; lane 3, Upper (PEG-rich) phase before washings; lane 4.- Lower (dextran-rich) phase before washing; lane 5.-Upper phase after the first washing; lane 6, Lower phase after the first washing. lane 7, Upper phase after the second washing. lane 8, Lower phase after the second washing. (B), lane 1, Molecular weight standard; lane 2, Upper phase after the first elution; lane 3, Lower phase after the first elution; lane 4, Upper phase after the second elution; lane 5, Lower phase after the second elution.

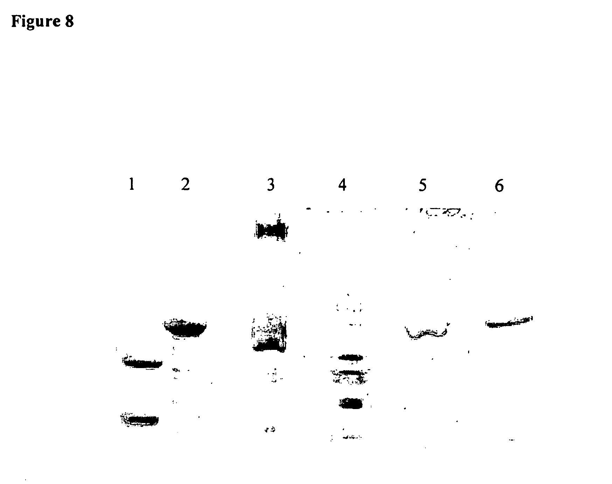

**Figure 8****.** SDS-PAGE of the fractions coming from the LYTAG-β-galactosidase purification in PEG/dextran. lane 1, Molecular weight standard; lane 2, Crude extract of REG-1 [pALEX2c-LacZ]; lane 3, Upper (PEG-rich) phase before washing; lane 4.- Lower (dextran-rich) phase before washing; lane 5.- Upper phase after the first elution; lane 6, Lower phase after the first elution.

### REALIZATION EXAMPLES OF THE INVENTION

### Example 1: Purification of a GFP-LYTAG fusion

The present example shows purification of a green-fluorescent protein (GFP) as a fusion to a CBM module, i.e. the LYTAG protein. For this example, expression of the GFP-LYTAG protein is carried out in 400 ml LB + ampicilin culture of the *E. coli* REG-1 strain (Biomedal, Spain, www.biomedal.com) transformed with plasmid pALEX2-Ca-GFP, a derivative of the pALEX2-Ca expression vector (Biomedal), said culture being incubated at 37°C before inducing expression of the GFP-LYTAG protein by adding salicylate to the medium, according to the recommendations for the "LYTAG kit" (Biomedal). The cells are harvested by centrifugation and resuspended in 20 ml of 20 mM potassium phosphate buffer pH 8.0 when the purification is carried out with the PEG/Phosphate system, or in Tris-HCl 20 mM pH 8.0 in the case of the PEG/Dextran system. The cells are then disrupted by sonication, the obtained lysate centrifuged to remove cell debris and the supernatant mixed with the appropriate amounts of PEG and potassium phosphate or dextran to obtain the ExtP and ExtD buffers aforementioned. The mix is then gently shaken (to avoid DNA fragmentation and its presence as contaminant in the final protein preparation in a beaker until the total solubilization of the system components, and centrifuged for 5 minutes at 12.000 rpm and room temperature (for more temperature-sensitive proteins centrifugation can be carried out at 4°C). The specific affinity of LYTAG to PEG determines partitioning of the GFP-LYTAG protein to the PEG-rich (upper) phase in the two systems tested (Figure 5A). The green colour in the upper phase shows that the GFP domain remains functional and therefore properly folded in the system. On the other hand, the majority of cell proteins partition to the lower (phosphate- or dextran- rich) phase, resulting in a purity grade of the GFP-LYTAG fusion in the upper phase already high (Fig. 5C). Choline addition to the Wash buffers causes the GFP-LYTAG protein elution to the lower phase (Figs 5B and 5C). Note, that the SDS-PAGE gels presented in these figures show an anomalous mobility of the proteins, due to the high salt and polymer concentrations used. Although protein elution is not complete in a first extraction (Fig. 5C, street 6), successive extractions by addition of fresh lower phase solutions containing choline, allow recovering of most of the GFP-LYTAG fusion present in the system (data not shown). For this particular LYTAG fusion, the PEG/phosphate system yields up to 5 mg of protein/liter of culture and a purity grade slightly higher than that achieved with the PEG/Dextran system.

### Example 2: Purification of a LYTAG-Protein A fusion

This example shows how the present invention can be applied to the purification of a LYTAG fusion to the immunoglobulin binding domain of *Staphylococcus aureus* protein A, by following the same procedures as those described in Example 1, in this case using a culture of the REG-1 strain transformed with the pALEXb-ProtA plasmid, a derivative of the pALEXb expression vector (Biomedal). The LYTAG-Protein A fusion is efficiently purified using both the PEG/Dextran (Figures 6A and 6B) and the PEG/Phosphate system (Figure 6C). As in Example 1, the PEG/Dextran system performance increases with additional washing steps. Moreover, after the washing steps the LYTAG fusion remains in the PEG-rich phase with a high purity grade and addition of choline allows quantitative recovering of the protein in the lower phase.

### Example 3: Purification of a LYTAG-Lip36 fusion

Purification of the LYTAG-Lip36 fusion protein is carried out as in the previous examples, using a culture of the strain REG-1 transformed with the pALEXb-Lip36 plasmid, a derivative of the pALEXb expression vector (Biomedal). In this case, the PEG/dextran system shows a higher efficiency, requiring additional washing steps as in the previous examples. Purification yields of this protein range between 5-10 mg per litre of culture.

### Example 4: Purification of a LYTAG-β-galactosidase fusion

Purification of the *LYTAG-β-galactosidase* fusion protein is carried out as the previous examples, using a culture of the strain REG-1 transformed with the pALEX2c-LacZ plasmid, a derivative of the pALEX2c expression vector (Biomedal), and this example shows the capacity of the system for partitioning and purifying an oligomeric, high molecular weight protein as the *E. coli* beta-galactosidase, whose biophysical features might interfere with the LYTAG-driven partitioning in the system. Results indicates that, in spite of significant accumulation of the fusion protein in the interphase region, it can be purified with an acceptable purity grade in the PEG/dextran system, as it is shown in the Figure 8. The purified protein retains its activity in assays with the synthetic substrate *o-*nitrophenylgalactopyranoside (ONPG) (data not shown), indicating proper folding and structural stability of the protein as in the case of the GFP-LYTAG fusion described in Example 1.

### Example 5: Purification of LYTAG fusion proteins secreted to the culture medium

In this case, the present invention can be used for the purification of recombinant proteins secreted into the culture medium, from bacteria, yeasts or other microorganisms. The yeast *Pichia pastoris* has an efficient secretion machinery, allowing production of proteins of interest fused to a leader peptide that directs its transport towards the extracellular medium, through, for example, the use of the pPIC9 vector (*Pichia* Expression Kit, Invitrogen). If this secreted protein is expressed as a LYTAG fusion, as those expressed from the the pPIC9-CLYT vector (Caubin, J., et al., Choline-binding domain as a novel affinity tag for purification of fusion proteins produced in Pichia pastoris. Biotechnol Bioeng, 2001. 74(2): p. 164-71), the resulting protein can be purified from the extracellular medium by using one of the aqueous two-phase systems described in this patent. With this aim, a transformant of the *P. pastoris* strain GS 115 is obtained using a pPIC9-CLYT plasmid derivative, expressing a secretable fusion of LYTAG to the protein of interest. This transformant can then be grown in BMGY buffered medium at 30 °C until a DO₆₀₀ of 2-6, and the expression of the secretable LYTAG fusion can be induced by switching the carbon source in the culture medium, from 1% glycerol (BMGY medium) to 0.5% methanol (BMMY medium). After 15-24 hours of induction, the culture is centrifuged and the supernatant, containing the fusion protein can be directly mixed with the components of one of the purification systems described in the previous examples, leading this process to the partitioning and purification of the protein of interest.

Likewise, the purification of LYTAG-fused proteins from the culture medium can be applied to *E. coli* cultures expressing LYTAG-protein or protein-LYTAG fusions to leader peptides that direct transport of the fusion proteins to the periplasmic space or the extracellular medium.

### Example 6: Purification of antibodies in two-phase aqueous systems through the use of LYTAG-protein A fusions

The protein A from *S. aureus* displays strong affinity to immunoglobulins, a feature that can be applied to the purification of antibodies using the two-phase systems described in the Example 2. In the present example, the two-phase components are dissolved in a mixture of 1 ml of LYTAG-Protein A (5-10 mg/ml) purified and dialized in 20 mM phosphate buffer pH 8.0 (or 20 mM Tris-HCl buffer pH 8.0 if the PEG/dextran system is used), and 19 ml of the supernatant of a hybridome culture expressing the antibody of interest and dialyzed in the same buffer. Once the two-phase system is generated, the procedures described in the previous examples are followed in order to recover the resulting LYTAG-protein A-antibody complexes in the lower phase, with a high purity grade. Alternatively, the antibodies can be separated from the LYTAG-protein A when the complexes are in the PEG-rich phase, by applying pH and ionic force conditions that disrupt the protein A-antibody interaction, and finally recovered in the lower phase, while being able to recycle the LYTAG-protein A fusion for further antibody purification processes.

## Claims

1. Procedure for partitioning or separating proteins or polypeptides linked to choline binding polypeptides **characterized by**:
a) contacting
i) a crude or partially purified extract of a cell culture, or the extracellular medium of a cell culture, or a mixture of different chemical compounds containing at least one protein or polypeptide whose sequence has at least one choline-binding structural motif,
ii) with a mixture of water, polyethylene glycol (PEG) and salts or other polymers more than 5% soluble in water,
the resulting mixture being spontaneously structured into a binary system with two aqueous phases, A and B, phase A being richer in PEG than phase B, and wherein proteins or polypeptides with said choline-binding motifs partition preferentially to phase A
b) optionally suppressing the preferential partitioning of said proteins or polypeptides containing the choline binding motifs to phase A, by adding a solution containing choline.

2. The procedure according to claim 1 **characterized in that** the total PEG concentration in the binary system is comprised between 3 and 15%.

3. The procedure according to any of claims 1 to 2, **characterized in that** the salt present in the phase B of the binary system is selected between a phosphate, citrate, sulphate salt or a mixture thereof.

4. The procedure according to any of claims 1 to 3, **characterized in that** the salt present in the phase B of the binary system contains potassium phosphate at a concentration comprised between 5 and 15%, and the pH of the mixture has a value comprised between 6.0 and 9.0.

5. The procedure according to any of claims 1 to 2, **characterized in that** the polymer present in the phase B of the binary system is dextran or a dextran derivative at a concentration comprised between 5 and 10%, and the pH of the mixture has a value comprised between 6.0 and 9.0.

6. The procedure according to claims 1 to 2, **characterized in that** the polymer present in the phase B of the binary system is starch or a starch derivative at a concentration comprised between 5 and 10% and the pH of the mixture has a value comprised between 6.0 and 9.0.

7. The procedure according to any of claims 1 to 6, **characterized in that** the polypeptidic sequence with affinity to choline derives from the C-terminal domain of the LytA amidase of *Streptococcus pneumoniae.*

8. The procedure according to any of claims 1 to 7, **characterized in that** the cell extract or the extracellular medium of the cellular culture originate from hybridomes, fungi, yeast or bacteria.

9. The procedure according to any of claims 1 to 8, **characterized in that** it comprises a further step consisting of the partial removal of the proteins from the cell extract or the extracellular culture medium that do not partition preferentially to phase A, by discarding phase B.

10. The procedure according to claim 9, **characterized in that** the proteins that do not partition preferentially to phase A are quantitatively eliminated through the following steps:
i) adding an amount of solution equivalent in composition and volume to those of the phase B discarded,
ii) mixing and separation in two phases, A and B
iii) discarding the newly formed phase B; and
iv) optionally repeating the previous steps.

11. The procedure according to any of claims 1 to 10, **characterized in that** the PEG-rich phase A containing the fusion protein or fusion polypeptide is used directly to obtain said fusion protein or fusion polypeptide in purified form.

12. The procedure according to any of claims 1-10 **characterized in that** the protein or polypeptide fused to the choline binding motif(s) is a catalyst and the resulting binary system can be used directly as a reactor, to catalyze a reaction of interest in which said catalyst remains in the PEG-rich phase A and the product(s) of the reaction can be recovered in the opposite, PEG-poor phase B.

13. Use of the C-terminal domain of the LytA amidase of *Streptococcus pneumoniae* as the sequence with affinity to choline for the partitioning or separation of fusion proteins or fusion polypeptides according to any of claims 1 to 11.

14. Use of a Kit comprising the following components to perform the procedure for partitioning or separation of fusion proteins or fusion polypeptides according to any of claims 1-11:
i) an aqueous solution with a PEG concentration between 3 and 15%;
ii) an aqueous solution comprising a phosphate, citrate or sulphate salt or a mixture thereof, or dextran or a dextran derivative, or starch or a starch derivative; and
iii) choline as a solid salt or in aqueous solution.

## Patentansprüche

1. Verfahren für die Verteilung oder Trennung von Proteinen oder Polypeptiden, die mit den Polypeptiden für die Bindung an Cholin verknüpft sind, **dadurch gekennzeichnet, dass**:
a) folgendes miteinander in Kontakt gebracht wird:
i) ein ungereinigter oder teilweise gereinigter Extrakt einer Zellkultur oder das extrazelluläre Medium einer Zellkultur, oder ein Gemisch verschiedener chemischer Verbindungen, die zumindest ein Protein oder Polypeptid enthalten, dessen Sequenz zumindest ein Strukturmotiv einer Bindung an Cholin aufweisen,
ii) mit einem Gemisch aus Wasser, Polyethylenglykol (PEG) und Salze und anderen löslichen Polymeren mit einer Konzentration von über 5 % in Wasser,
wobei sich das erhaltene Gemisch spontan in ein binäres System mit zwei wässrigen Phasen, A und B, aufteilt, wobei die Phase A mehr PEG enthält als die Phase B, weshalb sich die Proteine oder Polypeptide mit den genannten Bindungsmotiven an Cholin vorzugsweise in der Phase A verteilen;
b) und wahlweise die bevorzugte Verteilung der genannten Proteine oder Polypeptide mit den Bindungsmotiven an Cholin in der Phase A aufgehoben wird, indem eine Cholin enthaltende Lösung hinzugefügt wird.

2. Verfahren nach Anspruch 1, **dadurch gekennzeichnet, dass** die PEG-Konzentration im binären System zwischen 3 und 15% liegt.

3. Verfahren nach einem der Ansprüche 1 bis 2, **dadurch gekennzeichnet, dass** das in der Phase B des binären Systems vorhandene Salz gewählt wird aus einem Phosphat-, Zitrat-, Sulfatsalz oder aus einem Gemisch dieser Salze.

4. Verfahren nach einem der Ansprüche 1 bis 3, **dadurch gekennzeichnet, dass** das in der Phase B des binären Systems vorhandene Salz Kaliumphosphat mit einer Konzentration von 5 bis 15 % enthält und der pH des Gemisches einen Wert von 6,0 bis 9,0 aufweist.

5. Verfahren nach einem der Ansprüche 1 bis 2, **dadurch gekennzeichnet, dass** das in der Phase B des binären Systems vorhandene Polymer Dextran ist oder ein Dextranderivat mit einer Konzentration von 5 bis 10 % und der pH des Gemisches einen Wert von 6,0 bis 9,0 aufweist.

6. Verfahren nach den Ansprüchen 1 bis 2, **dadurch gekennzeichnet, dass** das in der Phase B des binären Systems vorhandene Polymer Stärke ist oder ein Stärkederivat mit einer Konzentration von 5 bis 10 % und der pH des Gemisches einen Wert von 6,0 bis 9,0 aufweist.

7. Verfahren nach einem der Ansprüche 1 bis 6, **dadurch gekennzeichnet, dass** die Polypeptidsequenz mit Affinität zu Cholin aus einer C-terminalen Domäne der Amidase LytA des *Streptococcus pneumoniae* gewonnen wird.

8. Verfahren nach einem der Ansprüche 1 bis 7, **dadurch gekennzeichnet, dass** der Zellextrakt oder das extrazelluläre Medium einer Zellkultur aus Hybridoma-Zellen, Pilzen, Hefen oder Bakterien stammen.

9. Verfahren nach einem der Ansprüche 1 bis 8, **dadurch gekennzeichnet, dass** dieses einen zusätzlichen Schritt umfasst, der darin besteht, die Proteine des Zellextrakts oder des extrazellulären Kulturmediums, die sich nicht bevorzugt in der Phase A ansammeln, teilweise zu entfernen, indem die Phase B verworfen wird.

10. Verfahren nach Anspruch 9, **dadurch gekennzeichnet, dass** die Proteine, die sich nicht bevorzugt in der Phase A verteilen, über folgende Schritte mengenmäßig entfernt werden:
i) Hinzugabe einer Menge an Lösung, die in der Zusammensetzung und im Volumen der verworfenen Phase B entspricht,
ii) Mischung und Trennung in zwei Phasen, A und B,
iii) Entfernung der neu gebildeten Phase B; und
iv) wahlweise die Wiederholung der beiden vorhergehenden Schritte.

11. Verfahren nach einem der Ansprüche 1 bis 10, **dadurch gekennzeichnet, dass** die Phase A, die mehr PEG enthält und die das Fusionsprotein oder das Fusionspolypeptid enthält, direkt benutzt wird, um das Fusionsprotein oder das Fusionspolypeptid in gereinigter Form zu erhalten.

12. Verfahren nach einem der Ansprüche 1 bis 10, **dadurch gekennzeichnet, dass** das Protein oder das Polypeptid, das mit dem Bindungsmotiv oder den -motiven an Cholin fusioniert, ein Katalysator ist und dass das erhaltene binäre System direkt als Reaktor verwendet werden kann, um die angestrebte Reaktion zu katalysieren, bei der der genannte Katalysator in der Phase A verbleibt, die viel PEG enthält und das oder die Reaktionsprodukt(e) aus der auf der anderen Seite erhaltenen Phase B, die wenig PEG enthält, wiedergewonnen werden kann/können.

13. Benutzung der C-terminalen Domäne der Amidase LytA des *Streptococcus pneumoniae* als Sequenz mit einer Affinität zu Cholin, für die Verteilung oder Trennung von Fusionsproteinen oder -polypeptiden nach einem der Ansprüche 1 bis 11.

14. Benutzung eines Kits, das folgende Bestandteile enthält, um das Verfahren für die Verteilung oder Trennung von Fusionsproteinen oder -polypeptiden nach einem der Ansprüche 1-11 durchzuführen:
i) eine wässrige Lösung mit einer PEG-Konzentration zwischen 3 % und 15 %;
ii) eine wässrige Lösung, umfassend ein Phosphat-, Zitrat-, Sulfatsalz oder ein Gemisch dieser Salze oder ein Dextran oder ein Dextranderivat, oder Stärke oder ein Stärkederivat; und
iii) Cholin in Form eines festen Salzes oder in einer wässrigen Lösung.

## Revendications

1. Procédé de distribution ou séparation de protéines ou polypeptides liés aux polypeptides de liaison à la choline **caractérisé en ce que**:
a) on a mis en contact
i) un extrait en brut ou partiellement purifié d'une culture cellulaire, ou le moyen extracellulaire d'une culture cellulaire, ou bien un mélange de plusieurs composés chimiques contenant au moins une protéine ou polypeptide dont la séquence possède, au moins, un motif structural de liaison à la choline,
ii) avec un mélange d'eau, de polyéthylène glycol (PEG) et de sels ou d'autres polymères solubles sur plus de 5% d'eau,
le mélange résultant se structure spontanément en un système binaire avec deux phases aqueuses, A et B, dans lesquelles la phase A est plus riche en PEG que la phase B et dans laquelle les protéines ou polypeptides avec ces motifs de liaison à la choline sont distribués de préférence dans la phase A;
b) on a supprimé optionnellement la distribution préférentielle de ces protéines ou polypeptides contenant des motifs de liaison à la choline dans la phase A, en ajoutant une solution qui contient de la choline.

2. Procédé selon la revendication 1, **caractérisé en ce que** la concentration de PEG dans le système binaire est comprise entre 3 et 15%.

3. Procédé selon l'une quelconque des revendications 1 et 2, **caractérisé en ce que** le sel présent dans la phase B du système binaire est sélectionné parmi un sel de phosphate, citrate, sulfate ou un mélange de ces derniers.

4. Procédé selon l'une quelconque des revendications 1 à 3 **caractérisé en ce que** le sel présent dans la phase B du système binaire contient du phosphate de potassium avec une concentration comprise entre 5 et 15% et où le pH du mélange a une valeur comprise entre 6,0 et 9,0.

5. Procédé selon l'une quelconque des revendications 1 à 2, **caractérisé en ce que** le polymère présent dans la phase B du système binaire est du dextrane ou un dérivé du dextrane avec une concentration comprise entre 5 et 10%, et où le pH du mélange a une valeur comprise entre 6,0 et 9,0.

6. Procédé selon l'une quelconque des revendications 1 à 2, **caractérisé en ce que** le polymère présent dans la phase B du système binaire est de l'amidon ou un dérivé de l'amidon avec une concentration comprise entre 5 et 10%, et où le pH du mélange a une valeur comprise entre 6,0 et 9,0.

7. Procédé selon l'une quelconque des revendications 1 à 6, **caractérisé en ce que** la séquence polypeptidique de liaison à la choline est obtenue à partir du domaine C-terminal de l'amidase LytA du *Streptococcus pneumoniae.*

8. Procédé selon l'une quelconque des revendications 1 à 7, **caractérisé en ce que** l'extrait cellulaire ou le milieu extracellulaire d'une culture cellulaire proviennent d'hybridomes, champignons, levures ou bactéries.

9. Procédé selon l'une quelconque des revendications 1 à 8, **caractérisé en ce qu'**il comprend une phase additionnelle qui consiste en l'élimination partielle des protéines de l'extrait cellulaire ou du milieu de culture cellulaire qui ne s'accumulent pas dans la phase A et en excluant la phase B.

10. Procédé selon la revendication 9, **caractérisé en ce que** les protéines ne sont pas distribuées dans la phase A et s'éliminent de façon quantitative à travers les phases suivantes:
i) addition d'une quantité de solution équivalente quant à la composition et au volume à la phase B exclue,
ii) mélange et séparation en deux phase, A et B,
iii) élimination de la phase B récemment formée; et
iv) optionnellement, la répétition des phases précédentes.

11. Procédé selon l'une quelconque des revendications 1 à 10, **caractérisé en ce que** la phase A riche en PEG contenant la protéine de fusion ou le polypeptide de fusion s'utilise directement pour obtenir cette protéine de fusion ou polypeptide de fusion d'une façon purifiée.

12. Procédé selon l'une quelconque des revendications 1 à 10, **caractérisé en ce que** la protéine ou le polypeptide fondu au motif ou aux motifs de liaison à la choline est un catalyseur et **en ce que** le système binaire résultant peut être directement utilisé comme un réacteur, pour catalyser une réaction d'intérêt dans laquelle ce catalyseur reste dans la phase A riche en PEG et le produit ou les produits de la réaction peuvent être récupérés dans la phase opposée, pauvre en PEG.

13. Utilisation du domaine C-terminal de l'amidase LytA du *Streptococcus pneumoniae* en tant que séquence analogue à la choline pour la distribution ou séparation des protéines ou polypeptides de fusion selon l'une quelconque des revendications 1 à 11.

14. Utilisation d'un kit comprenant les composants suivants pour la réalisation du procédé de distribution ou de séparation des protéines ou polypeptides de fusion selon l'une quelconque des revendications 1-11:
i) une solution aqueuse d'une concentration en PEG allant de 3% à 15%;
ii) une solution aqueuse comprenant du sel de phosphate, citrate, sulfate ou un mélange de ces derniers, ou du dextrane ou un dérivé du dextrane, ou de l'amidon ou un dérivé de l'amidon ; et
iii) choline sous forme de sel solide ou dans une solution aqueuse.
